# EUROPEAN PATENT SPECIFICATION

(11) **EP 1 304 562 B1**
(45) Date of publication and mention of the grant of the patent: **28.06.2006**
(21) Application number: 01125092.5
(22) Date of filing: 22.10.2001
(51) Int. Cl.: G01N 23/04

(54) **X-ray inspection system for products, in particular food products**
Röntgenstrahlung-Inspektionssystem für Produkte, insbesondere Nahrungsmittel
Dispositif d'inspection à rayons-x pour produits, notamment produits alimentaires

(43) Date of publication of application: 23.04.2003
(73) Proprietor: Miconos Technology Limited, Valletta (MT)
(72) Inventor: Toso, Antonio, 10124 Torino (IT)
(74) Representative: Bongiovanni, Simone

(56) References cited:
- WO-A-01/22071
- GB-A- 2 285 506
- US-A- 3 488 495
- US-A- 5 981 963

## Description

The present invention relates to an x-ray inspection system for products, in particular food products.

X-ray inspection systems for products, in particular food products, are known in which a conveying system, e.g. a conveyor belt, feeds the products into an inspection position in which an x-ray generating device directs an x-ray beam on to the moving product, and a sensor device, in particular a linear sensor, receives the x-rays which have traveled through the product to generate a radiographic image of the product.

The radiographic image is processed automatically to determine any flaws and/or changes in the product or product packaging.

In known systems, the x-ray generating and sensor devices are fixed firmly to the conveying system, in particular, are housed inside a supporting structure of the conveying system, so that to repair, check, or change any parts on the generating and/or sensor device, the conveying system must be stopped and at least partly dismantled to gain access to the devices.

This is a time-consuming, painstaking job which necessarily involves stopping the conveyor system.

Document WO-A-0 122 071 describes a x-ray inspection system as detailed in the preamble of claim 1.

It is an object of the present invention to provide an x-ray inspection system for products, in particular food products, designed to eliminate the drawbacks of known systems.

According to the present invention, there is provided an x-ray inspection system for products, in particular food products, as claimed in Claim 1.

A preferred, non-limiting embodiment of the invention will be described by way of example with reference to the accompanying drawings, in which:
Figure 1 shows a front view of an x-ray inspection system for products, in particular food products, in accordance with the teachings of the present invention;
Figure 2 shows an inspection unit of the Figure 1 inspection system;
Figure 3 shows a further embodiment of the inspection system according to the present invention.

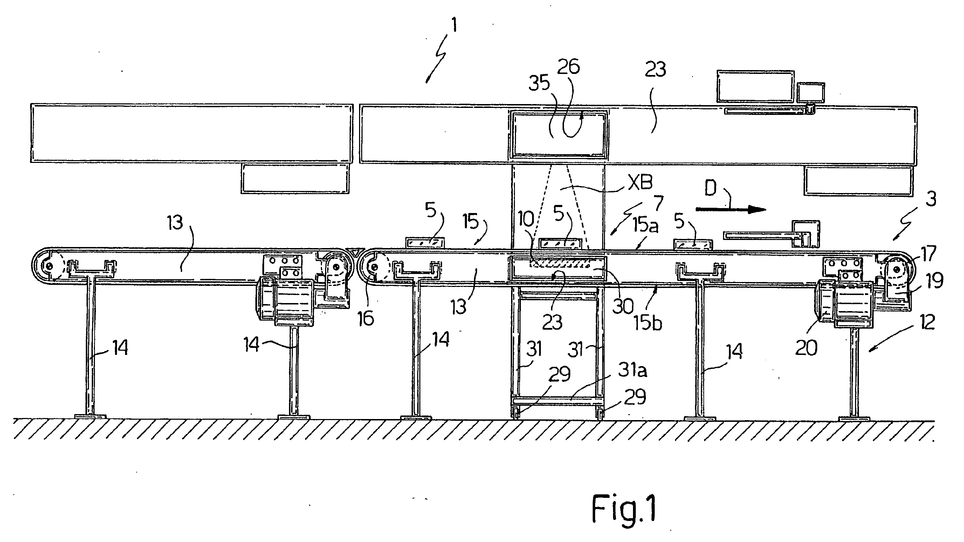

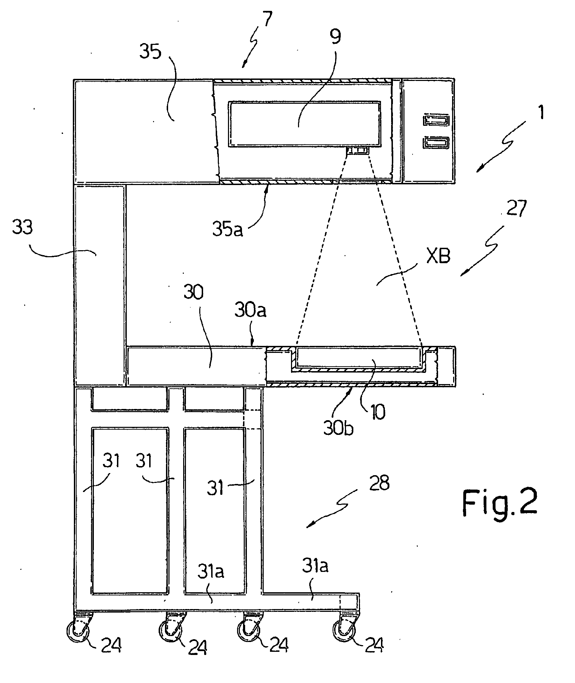

Number 1 in Figures 1 and 2 indicates as a whole an x-ray inspection system for products, in particular food products.

Inspection system 1 comprises a conveying system 3 for feeding a number of products 5 in a traveling direction D; and at least one inspection unit 7, which is movable with respect to, and can be connected/disconnected to/from, conveying system 3.

Products 5 may comprise a number of different products, such as:
- liquid food products (e.g. water, alcohol, fruit juice, and drinks in general) in containers made of any material (glass, plastic, paper or metal);
- semiliquid or gelatinous food products (sauce, cream, jelly or jam);
- solid food products in general, either loose or in containers.

The system according to the present invention obviously also operates perfectly well with any type of other than food product (e.g. drugs, dietetic products) whose internal characteristics can be x-ray inspected.

Moreover, traveling direction D may obviously differ, e.g. be curved, as opposed to straight as in the example shown.

Inspection unit 7 (Figure 2) comprises at least one known x-ray generating device 9 and at least one known x-ray sensor device 10 - in particular, a linear sensor - spaced apart and facing each other.

Generating device 9 produces an x-ray beam XB, which strikes products 5 traveling in direction D (Figure 1) when inspection unit 7 is connected to conveying system 3.

More specifically, conveying system 3 is a conveyor belt type, and comprises a supporting structure 12 defined by a horizontal bed 13 and by a number of vertical members 14 supporting bed 13.

Supporting structure 12 supports a conveyor belt 15 extending between two transmission rollers 16, 17 at opposite portions of bed 13. More specifically, roller 16 is idle, while roller 17 is driven, via a transmission 19, by an electric motor 20 fitted to bed 13.

Conveyor belt 15 defines a straight, flat top portion 15a supporting products 5, and a straight bottom return portion 15b; and bed 13 defines a first parallelepiped-shaped through cavity 23 extending crosswise to direction D, located between portions 15a and 15b, and open at opposite sides.

A horizontal top structure 23 is fitted over bed 13, extends at a given height from conveyor belt 15, is fitted to vertical spacer members (not shown) extending from bed 13, and defines a second parallelepiped-shaped through cavity 26 extending crosswise to direction D and open at opposite sides.

Cavities 23 and 26 are substantially the same width and are positioned facing each other.

Inspection unit 7 comprises a C-shaped main body 27 supporting generating device 9 and sensor device 10; and a trolley 28 mounted on wheels 29 and supporting main body 27.

More specifically, main body 27 comprises a substantially parallelepiped-shaped base body 30 defined, on opposite sides, by a flat rectangular top wall 30a, and by a rectangular bottom wall 30b, from which straight members 31 extend at a first portion of body 30, and are fitted on the ends with horizontal cross members 31a supporting wheels 29. Base body 30 houses sensor device 10, which is located close to wall 30a, in a second portion of body 30.

Main body 27 also comprises a connecting body 33 connected to a lateral wall of base body 30 and extending perpendicular to wall 30a.

Main body 27 also comprises a top body 35 projecting from the top end of body 33 and facing and spaced apart from base body 30. More specifically, top body 35 is defined, among other things, by a flat rectangular wall 35a facing and parallel to wall 30a, and houses x-ray generating device 9, which directs x-ray beam XB through the space between top body 35 and base body 30.

In actual use, to connect inspection unit 7 to conveying system 3, inspection unit 7 is positioned with base body 30 facing an opening in parallelepiped-shaped cavity 23, and with top body 35 facing a corresponding opening in cavity 26.

Inspection unit 7 is then pushed in a direction crosswise to direction D towards conveying system 3 so that base body 30 penetrates parallelepiped-shaped cavity 23, and top body 35 penetrates cavity 26.

Sensor device 10 is thus positioned beneath straight top portion 15a, and x-ray generating device 9 is positioned facing conveyor belt 15 and over straight top portion 15a.

Conveying system 3 is then supplied at the input with a number of products 5 for inspection. More specifically, products 5 are fed (e.g. by means of a further conveying system shown in Figure 1) to an initial portion of conveyor belt 15, which feeds them in direction D to inspection unit 7. Each product 5 therefore travels in an inspection position, in which product 5 resting on belt 15 is positioned over sensor device 10 and facing x-ray generating device 9, by which x-ray beam XB is directed on to and through product 5 to generate a radiographic image of the product by means of sensor 10. The radiographic image of the product is picked up and processed in known manner to determine any flaws and/or changes in the product or product package.

On reaching the end of conveyor belt 15, products 5 are fed to other conveying devices or removed by gripping devices.

When inspection and/or cleaning is required, inspection unit 7 can be disconnected easily from conveying system 3 by pushing the inspection unit crosswise to direction D away from conveying system 3, to expel base body 30 from parallelepiped-shaped cavity 23, and top body 35 from cavity 26.

Once removed from conveying system 3, all the external component parts of inspection unit 7 are accessible for easy cleaning and/or inspection.

To repair, change any parts of, or check generating device 9 and/or sensor device 10, therefore, no work is required on conveying system 3, which need not be arrested and can therefore continue conveying the products, and no parts of conveying system 3 need be disassembled; and inspection unit 7 can be reconnected to conveying system 3 quickly and easily.

Maintenance of the inspection system is thus greatly improved.

Conveying system 3 may undergo routine cleaning (especially when product 5 is a loose, highly perishable product such as meat, particularly white or minced meat) using detergents and disinfectants which are normally highly aggressive with respect to sensor device 10 and generating device 9.

According to the present invention, however, to clean conveying system 3, inspection unit 7 is removed from conveying system 3 as described above, so that cleaning, washing or disinfecting conveying system 3 has no effect on generating device 9 or sensor device 10.

Clearly, changes may be made to the x-ray inspection system for products, in particular food products, as described and illustrated herein, without, however, departing from the scope of the present invention.

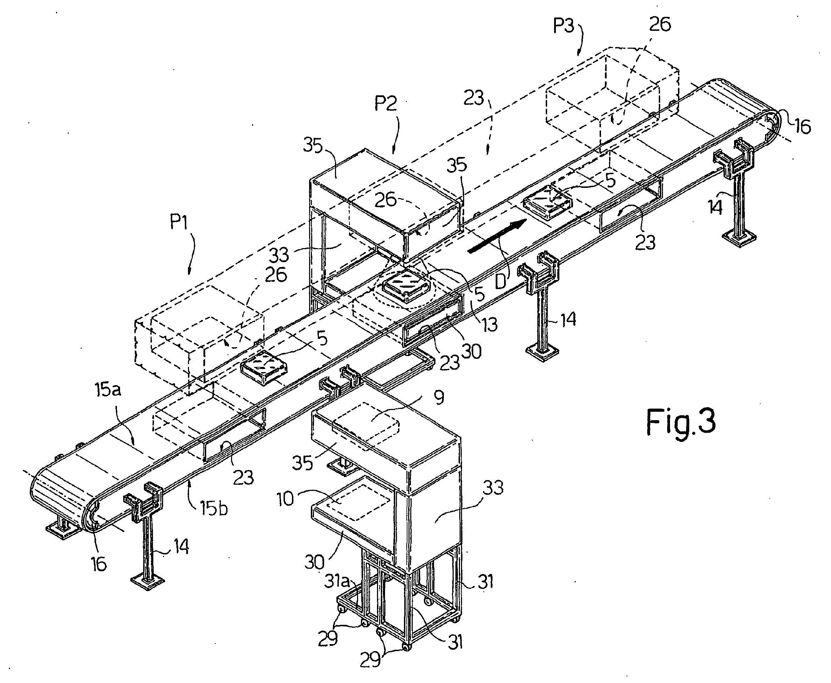

For example, the Figure 3 inspection system comprises a number of pairs of cavities 23, 26 spaced apart in direction D at successive points (indicated P1, P2, P3 in Figure 3) along bed 13 of conveying system 3, so that the same inspection unit 7, and therefore sensor device 10 and generating device 9, may be located selectively at different points along conveying system 3. Alternatively, conveying system 3 may be connected to a number of inspection units 7.

## Claims

1. An x-ray inspection system for products, in particular food products, comprising a conveying system (3) for feeding a number of products (5) in a traveling direction (D); and at least one inspection unit (7) having at least one x-ray generating device (9) and at least one x-ray sensor device (10);
said inspection unit (7) is movable (29) with respect to the conveying system (3), and can be connected/disconnected to/from the conveying system (3); the inspection unit (7) producing an x-ray beam (XB), which strikes the products (5) moving in the traveling direction (D), when the inspection unit (7) is connected to the conveying system (3).
**characterized in that** said conveying system (3) comprises a supporting structure (13) having at least one first cavity (23);
said inspection unit (7) comprising at least one base body (30) supporting said x-ray sensor device (10); and said base body (30) penetrating said first cavity (23) to connect said inspection unit (7) to said conveying system (3);
said x-ray beam (XB) striking the products (5) moving in the traveling direction (D), and being received by said x-ray sensor device when the inspection unit (7) is connected to the conveying system (3);
said supporting structure (13) comprises a second cavity (26);
said inspection unit (7) comprising a top body (35) supporting said x-ray generating device (9); and said top body (35) penetrating said second cavity (26), when said inspection unit (7) is connected to said conveying system (3), so that said x-ray generating device (9) is positioned facing the products moving along said conveying system.

2. A system as claimed in Claim 1, wherein said inspection unit (7) comprises a C-shaped main body (27);
said main body (27) comprising:
- said base body (30);
- a connecting body (33) extending crosswise to said base body (30); and
- said top body (35) projecting from said connecting body (33).

3. A system as claimed in claim 1 or 2, wherein said inspection unit (7) is movable on wheels (29).

4. A system as claimed in any one of the foregoing Claims, wherein said conveying system is a conveyor belt system.

5. A system as claimed in Claim 1, wherein said conveying system comprises a conveyor belt (15) defining a straight top first portion (15a) on which said products (5) are placed, and a bottom return portion (15b);
said first cavity (23) extending between said first portion (15a) and said bottom return portion (15b) of said belt (15).

6. A system as claimed in Claim 1, wherein said conveying system (3) comprises a supporting structure (13) having a number of first cavities (23) spaced in a traveling direction of the conveying system;
said inspection unit (7) comprising at least one base body (30) supporting said x-ray sensor device (10); said base body (30) being inserted selectively in one of said first cavities (23) to connect said inspection unit (7) to said conveying system (3) so that said x-ray generating device (9) and said x-ray sensor device (10) are located at a predetermined point (P1, P2, P3) along said conveying system.

## Patentansprüche

1. Röntgenstrahlung-Inspektionssystem für Produkte, insbesondere Nahrungsmittel, umfassend ein Fördersystem (3) zum Fördern einer Anzahl von Produkten (5) in einer Förderrichtung (D); und mindestens eine Inspektionseinheit (7) mit mindestens einer Röntgenstrahlung erzeugenden Vorrichtung (9) und mindestens einer Röntgenstrahlungssensorvorrichtung (10);
wobei die Inspektionseinheit (7) bewegbar (29) bezüglich des Fördersystems (3) ist und verbunden/getrennt mit/von dem Fördersystem (3) sein kann; wobei die Inspektionseinheit (7) einen Röntgenstrahl (XB) erzeugt, der die in Förderrichtung (D) bewegten Produkte (5) überstreicht, wenn die Inspektionseinheit (7) mit dem Fördersystem (3) verbunden ist,
**dadurch gekennzeichnet, daß** das Fördersystem (3) eine Tragkonstruktion (13) mit mindestens einem ersten Hohlraum (23) aufweist;
wobei die Inspektionseinheit (7) mindestens einen Grundkörper (30) aufweist, der die Röntgensensorvorrichtung (10) trägt; und der Grundkörper (30) den ersten Hohlraum (23) durchdringt, um die Inspektionseinheit (7) mit dem Fördersystem (3) zu verbinden;
wobei der Röntgenstrahl (XB) die in Förderrichtung (D) bewegten Produkte (5) überstreicht und durch die Röntgensensorvorrichtung empfangen wird, wenn die Inspektionseinheit (7) mit dem Fördersystem (3) verbunden ist;
wobei die Tragkonstruktion (13) einen zweiten Hohlraum (26) aufweist;
wobei die Inspektionseinheit (7) einen oberen die Röntgenstrahlung erzeugende Vorrichtung (9) tragenden Körper (35) aufweist; und der obere Körper (35) den zweiten Hohlraum (26) durchdringt, wenn die Inspektionseinheit (7) mit dem Fördersystem (3) verbunden ist, so daß die Röntgenstrahlung erzeugende Vorrichtung (9) auf die entlang des Fördersystems bewegten Produkte hin ausgerichtet ist.

2. System nach Anspruch 1, bei dem die Inspektionseinheit (7) einen C-förmigen Hauptkörper (27) aufweist;
wobei der Hauptkörper (27) umfaßt:
- den Grundkörper (30);
- einen Verbindungskörper (33), der sich quer zum Grundkörper (30) erstreckt; und
- der obere Körper (35) von dem Verbindungskörper (33) vorsteht.

3. System nach Anspruch 1 oder 2, bei dem die Inspektionseinheit (7) auf Rädern (29) beweglich ist.

4. System nach einem der vorhergehenden Ansprüche, bei dem das Fördersystem ein Förderbandsystem ist.

5. System nach Anspruch 1, bei dem das Fördersystem ein Förderband (15) aufweist, das einen ersten geraden Abschnitt (15a), auf dem die Produkte (5) angeordnet sind, und einen unteren rückkehrenden Abschnitt (15b) aufweist;
wobei sich der erste Hohlraum (23) zwischen dem ersten Abschnitt (15a) und dem unteren rückkehrenden Abschnitt (15b) des Bands (15) erstreckt.

6. System nach Anspruch 1, bei dem das Fördersystem (3) eine Tragstruktur (13) aufweist, die eine Anzahl von ersten in Förderrichtung des Fördersystems beabstandeten Hohlräumen (23) aufweist;
wobei die Inspektionseinheit (7) mindestens einen eine Röntgensensorvorrichtung (10) tragenden Grundkörper (30) aufweist; wobei der Grundkörper (30) wahlweise in einen der ersten Hohlräume (23) einsetzbar ist, um die Inspektionseinheit (7) mit dem Fördersystem (3) zu verbinden, so daß die Röntgenstrahlung erzeugende Vorrichtung (9) und die Röntgensensorvorrichtung (10) an einem vorbestimmten Punkt (P1, P2, P3) entlang des Fördersystems angeordnet sind.

## Revendications

1. Système d'inspection aux rayons X pour produits, en particulier produits alimentaires, comprenant un système de convoyage (3) pour amener un nombre de produits (5) selon un sens de cheminement (D) ; et au moins une unité d'inspection (7) présentant au moins un dispositif générateur de rayons X (9) et au moins un dispositif capteur de rayons X (10) ;
l'unité d'inspection (7) étant déplaçable (29) par rapport au système de convoyage (3) et pouvant être connectée/déconnectée du système de convoyage (3), l'unité d'inspection (7) produisant un faisceau (XB) de rayons X, qui frappe les produits (5) se déplaçant dans le sens de cheminement (D) quand l'unité d'inspection (7) est connectée au système de convoyage (3),
**caractérisé en ce que** le système de convoyage (3) comprend une structure support (13) présentant au moins une première cavité (23) ;
l'unité d'inspection (7) comprenant au moins un corps de base (30) supportant le dispositif capteur de rayons X (10), et le corps de base (30) pénétrant dans la première cavité (23) pour connecter l'unité d'inspection (7) au système de convoyage (3) ;
le faisceau (XB) de rayons X frappant les produits (5) se déplaçant dans le sens de cheminement (D) et étant reçu par le dispositif capteur de rayons X quand l'unité d'inspection (7) est connectée au système de convoyage (3) ;
la structure support (13) comprend une deuxième cavité (26) ;
l'unité d'inspection (7) comprenant un corps supérieur (35) supportant le dispositif générateur de rayons X (9), et le corps de base (35) pénétrant dans la deuxième cavité (26) quand l'unité d'inspection (7) est connectée au système de convoyage (3), de telle sorte que le dispositif générateur de rayons X (9) est positionné en face des produits se déplaçant le long du système de convoyage.

2. Système selon la revendication 1, dans lequel l'unité d'inspection (7) comprend un corps principal en forme de C (27), le corps principal (27) comprenant :
- le corps de base (30) ;
- un corps de liaison (33) s'étendant en travers par rapport au corps de base (30) ; et
- le corps supérieur (35) se projetant depuis le corps de liaison (33)

3. Système selon l'une des revendications 1 ou 2, dans lequel l'unité d'inspection (7) est déplaçable sur des roues (29).

4. Système selon l'une des revendications précédentes, dans lequel le système de convoyage est un système de convoyage à courroie.

5. Système selon la revendication 1, dans lequel le système de convoyage comprend une courroie de convoyage (15) définissant une première partie supérieure droite (15a) sur laquelle sont placés les produits (5) et une partie de retour inférieure (15b),
la première cavité (23) s'étendant entre la première partie (15a) et la deuxième partie de retour inférieure (15b) de la courroie (15).

6. Système selon la revendication 1, dans lequel le système de convoyage (3) comprend une structure support (13) présentant un nombre de premières cavités (23) espacées selon le sens de cheminement du système de convoyage ;
l'unité d'inspection (7) comprenant au moins un corps de base (30) supportant un dispositif capteur de rayons X (10), le corps de base (30) étant inséré sélectivement dans l'une des premières cavités (23) pour connecter l'unité d'inspection (7) au système de convoyage (3), de telle sorte que le dispositif générateur de rayons X (9) et le dispositif capteur de rayons X (10) sont placés en un point déterminé (P1, P2, P3) le long du système de convoyage.
